# EUROPEAN PATENT APPLICATION

(11) **EP 4 023 229 A1**
(43) Date of publication of application: **06.07.2022**
(21) Application number: 20856954.1
(22) Date of filing: 31.08.2020
(51) Int. Cl.: A61K 31/713, C12N 15/09

(54) **SIRNA CAPABLE OF INHIBITING EXPRESSION OF MCM7 GENE, COMPOSITION, AND APPLICATION THEREOF**

(30) Priority: 30.08.2019 CN 201910816589
(71) Applicant: EnKang Pharmaceuticals (Guangzhou), Ltd., Guangzhou, Guangdong 510005 (CN); Foshan Intelgen Pharmaceutical Co. Ltd., Nanhai, Foshan, Guangdong 528200 (CN)
(72) Inventor: LIANG, Chun, Guangzhou, Guangdong 510005 (CN); WANG, Jun, Guangzhou, Guangdong 510005 (CN); CHUENG, Man Hei, Guangzhou, Guangdong 510005 (CN)
(74) Representative: Bayramoglu et al.
(86) International application number: PCT/CN2020/112563
(87) International publication number: WO 2021/037265

(57) **Abstract**

The present disclosure discloses a small interfering RNA (siRNA) for inhibiting the expression of the mini-chromosome maintenance 7 (MCM7) gene, and a composition and use thereof. The siRNA designed and verified by the present disclosure can effectively inhibit the expression of the MCM7 gene, and thus inhibit the DNA synthesis, cell proliferation, and colony formation of cancer cells, thereby achieving the purpose of preventing and treating a tumor. The present disclosure provides a new target and candidate compound for cancer prevention or treatment.

SEQUENCE LISTING

<110>  ENKANG PHARMACEUTICALS (GUANGZHOU), LTD.
       FOSHAN INTELGEN PHARMACEUTICALS CO., LTD.

<120>  SMALL INTERFERING RNA (siRNA) FOR INHIBITING EXPRESSION OF
       MINI-CHROMOSOME MAINTENANCE 7 (MCM7) GENE, AND COMPOSITION AND
       USE THEREOF

<130>  GBEKYY004-EPG

<150>  PCT/CN2020/112563
<151>  2020-08-31

<150>  CN 201910816589.5
<151>  2019-08-30

<160>  10

<170>  PatentIn version 3.5

<210>  1
<211>  19
<212>  RNA
<213>  Artificial sequence

<220>
<223>  siRNA-1 sense strand

<400>  1
guggagaauu gaccuuaga                                                    19


<210>  2
<211>  19
<212>  RNA
<213>  Artificial sequence

<220>
<223>  siRNA-1 antisense strand

<400>  2
ucuaagguca guucuccac                                                    19


<210>  3
<211>  19
<212>  RNA
<213>  Artificial sequence

<220>
<223>  siRNA(NC) sense strand

<400>  3
cucuuagcca auauucgcu                                                    19


<210>  4
<211>  19
<212>  RNA
<213>  Artificial sequence

<220>

<223>  siRNA(NC) antisense strand

<400>  4
agcgaauauu ggcuaagag                                                    19


<210>  5
<211>  20
<212>  DNA
<213>  Artificial sequence

<220>
<223>  Upstream primer

<400>  5
gtgaaggatc ctgcgacaca                                                   20


<210>  6
<211>  20
<212>  DNA
<213>  Artificial sequence

<220>
<223>  Downstream primer

<400>  6
acacgcgttc ttttgttccg                                                   20


<210>  7
<211>  24
<212>  DNA
<213>  Artificial sequence

<220>
<223>  Internal reference upstream primer

<400>  7
agaagagcta cgagctgcct gacg                                              24


<210>  8
<211>  22
<212>  DNA
<213>  Artificial sequence

<220>
<223>  Internal reference downstream primer

<400>  8
ggactccatg cccaggaagg aa                                                22


<210>  9
<211>  19
<212>  RNA
<213>  Artificial sequence

<220>
<223>  siRNA-1 sense strand

<400>  9

guggagaauu gaccuuaga 19

<210> 10
<211> 19
<212> RNA
<213> Artificial sequence

<220>
<223> siRNA-1 antisense strand

<400> 10
ucuaagguca guucuccac 19

## Description

### TECHNICAL FIELD

The present disclosure belongs to the field of biomedicine, and particularly relates to a small interfering RNA (siRNA) for inhibiting the expression of the mini-chromosome maintenance 7 (MCM7) gene, and a composition and use thereof.

### BACKGROUND

The mini-chromosome maintenance (MCM) complex consists of MCM2 to MCM7 subunits, which shows helicase activity in cells, and can unwind a DNA double strand before DNA replication and participate in the initiation of DNA replication (Bik Tye, Annual Review of Biochemistry, 1999). Moreover, the MCM complex also plays an important role in the regulation of cell proliferation, DNA damage repair, and the cell cycle.

siRNA is a double-stranded RNA with a length of 20 to 25 nucleotides, which was first discovered in the phenomenon of post-transcriptional gene silencing (PTGS) in plants. It has been publicly reported that synthetic siRNA can silence specific gene expression in mammalian cells (Thomas Tuschl et al., Nature, 2001; Thomas Tuschl et al., Science, 2001; Thomas Tuschl et al., Cell, 2002). Since siRNA can target interference at a genetic level without relying on crystal structures of the target proteins, scientists have studied a series of methods to inhibit the expression of target genes through RNA interference (RNAi), such as to obtain methods for gene function research and gene therapy.

Because different sequences at different sites on a target gene have different secondary structures (binary outcomes) and different thermodynamic properties, the possibility and degree of interference by siRNA at different sites will vary greatly. In addition, the same siRNA may show different activities in different types of cells. Therefore, for any target gene in any cell, the design, test, and acquisition of highly-active siRNA is a process of exploration and invention.

At present, there is no report on siRNA for inhibiting the MCM7 gene of cancer cells such as liver cancer, gastric cancer, and prostate cancer cells.

### SUMMARY

A first objective of the present disclosure is to provide a siRNA for inhibiting the expression of MCM7.

A second objective of the present disclosure is to provide use of the siRNA.

A third objective of the present disclosure is to provide a method for treating cancer by specifically targeting an MCM7 protein with siRNA.

The inventors have designed and tested many RNA interference fragments or the MCM7 gene, but most of the siRNAs have low interfering efficiency and cannot be effectively used for later tumor treatment research. Through creative exploration and research, the inventors have invented some efficient siRNA sequences for interfering with the MCM7 gene. This is crucial for the use of RNA interference, that is, the effects of siRNA against different sites of a target gene are very different, which may be related to the secondary (binary) structure and thermodynamic properties of siRNA, the free energy levels at the two termini, the base distribution, and other factors.

The inventors have found through further exploration that one or more bases of a sense strand of the siRNA can be changed, such that the sense strand and the antisense strand form an incomplete complementary pairing and the thermodynamic properties of the entire double-stranded RNA are changed, which improves the efficiency of the antisense strand to participate in the interference of RNA with a complex protein and thus improves the efficiency of the siRNA to inhibit the target MCM7 gene.

In order to achieve the above objectives, the present disclosure adopts the following technical solutions:
The inventors have designed a siRNA targeting the MCM7 gene. The siRNA inhibits the synthesis of MCM7 protein by inhibiting the expression of the human MCM7 gene, and thus blocks the formation of the entire MCM complex (MCM2-MCM7), thereby inhibiting DNA replication and cell proliferation to achieve the purpose of cancer prevention or treatment.

The formation of the complex can be inhibited by inhibiting the expression of any subunit of the MCM complex through siRNA, such as to inhibit cell proliferation and produce an anti-tumor effect.

In a first aspect of the present disclosure, a siRNA is provided, where the siRNA is capable of inhibiting the expression of the MCM7 gene, and is composed of a sense strand and an antisense strand; and
the siRNA is selected from one of the groups consisting of:
siRNA-1: sense strand: 5'-GUGGAGAAUUGACCUUAGA-3' (SEQ ID NO: 9), and
   antisense strand: 5'-UCUAAGGUCAGUUCUCCAC-3' (SEQ ID NO: 10);
a siRNA whose sense strand or antisense strand sequence has 80% or more homology or preferably 90% or more homology with the sense strand or antisense strand sequence of the siRNA-1; and a siRNA whose one or more nucleotides are able to be modified or changed by a conventional method in the prior art and which has the same or similar function and activity as or to the siRNA-1, where for example, 2'-OH in one or more nucleotides is changed into 2'-methoxy and a central oxygen atom of the phosphate is substituted by sulfur; or the sense strand or the antisense strand is added with cholesteryl.

Further, two deoxyribonucleotides dT or dN in a single-strand overhang structure need to be added to 3' termini of the sense strand and the antisense strand of the siRNA-1.

Further, the siRNA can prevent or treat a disease by inhibiting the expression of the MCM7 gene, and the disease may preferably be a tumor/cancer.

Further, the tumor/cancer may be selected from the group consisting of liver cancer, gastric cancer, prostate cancer, breast cancer, lung cancer, pancreatic cancer, cervical cancer, endometrial cancer, colorectal cancer, lung cancer, nasopharyngeal cancer, ovarian cancer, skin cancer, esophageal cancer, and brain tumor.

Further, the tumor may show slowed or stopped growth, shrink, or die due to the inhibition of the expression of the MCM7 gene.

Further, the MCM7 gene may be a human MCM7 gene.

Further, the siRNA sequence can be modified or changed at some or all of its nucleotide sites, for example, 2'-OH can be changed into 2'-methoxy and a central oxygen atom of the phosphate can be substituted by sulfur, or the sense strand or antisense strand can be added with cholesteryl, as long as the binding and inhibition of the target are not affected.

In a second aspect of the present disclosure, use of the siRNA in the preparation of a drug or a composition for preventing or treating a tumor/cancer is provided.

Further, the tumor/cancer may be selected from the group consisting of liver cancer, gastric cancer, prostate cancer, breast cancer, lung cancer, pancreatic cancer, cervical cancer, endometrial cancer, colorectal cancer, lung cancer, nasopharyngeal cancer, ovarian cancer, skin cancer, esophageal cancer, and brain tumor.

Further, the tumor/cancer may show slowed or stopped growth, shrink, or die due to the inhibition of the expression of the MCM7 gene.

Further, in the drug or composition, the siRNA may have a concentration of 5 nM to 150 nM, preferably 10 nM to 100 nM, more preferably 15 nM to 60 nM, and most preferably 20 nM to 40 nM.

There can also be the following additional technical features:
The siRNA can inhibit the expression of an MCM7 protein, and is composed of a sense strand and an antisense strand; and
the siRNA is selected from one of the groups consisting of:
   siRNA-1: sense strand: 5'-GUGGAGAAUUGACCUUAGA-3' (SEQ ID NO: 9), and
      antisense strand: 5'-UCUAAGGUCAGUUCUCCAC-3' (SEQ ID NO: 10);
   a siRNA whose sense strand or antisense strand sequence has 80% or more homology or preferably 90% or more homology with the sense strand or antisense strand sequence of the siRNA-1; and a siRNA whose one or more nucleotides are able to be modified or changed by a conventional method in the prior art and which has the same or similar function and activity as or to the siRNA-1, where for example, 2'-OH is changed into 2'-methoxy and a central oxygen atom of the phosphate is substituted by sulfur; or the sense strand or the antisense strand is added with cholesteryl. Further, two deoxyribonucleotides dT or dN in a single-strand overhang structure need to be added to 3' termini of the siRNA.

Further, the MCM7 gene may be a human MCM7 gene.

Further, the siRNA can be modified or changed at some or all of its nucleotide sites, for example, 2'-OH can be changed into 2'-methoxy and a central oxygen atom of the phosphate can be substituted by sulfur, or the sense strand or antisense strand can be added with cholesteryl, as long as the binding and inhibition of the target are not affected.

In a third aspect of the present disclosure, a drug or composition for preventing or treating a tumor/cancer is provided, comprising:
the siRNA described above;
an expression system capable of expressing the siRNA described above, such as a short hairpin RNA (shRNA) expression system.

Further, the tumor/cancer may be selected from the group consisting of liver cancer, gastric cancer, prostate cancer, breast cancer, lung cancer, pancreatic cancer, cervical cancer, endometrial cancer, colorectal cancer, lung cancer, nasopharyngeal cancer, ovarian cancer, skin cancer, esophageal cancer, and brain tumor.

Further, the tumor may show slowed or stopped growth, shrink, or die due to the inhibition of the expression of the MCM7 gene.

Further, in the drug or composition, the siRNA may have a concentration of 5 nM to 150 nM, preferably 10 nM to 100 nM, more preferably 15 nM to 60 nM, and most preferably 20 nM to 40 nM.

Further, the above drug or composition may also include:
a pharmaceutically acceptable carrier and/or adjuvant; and/or
other active ingredients for preventing or treating the tumor.

Further, the pharmaceutically acceptable carrier and/or adjuvant include(s), but are/is not limited to a buffering agent, an emulsifying agent, a suspending agent, a stabilizer, a preservative, a physiological salt, an excipient, a filler, a coagulating agent, a conditioner, a surfactant, a diffusing agent, and a defoaming agent.

Further, the other active ingredients for preventing or treating the tumor may include a chemotherapeutic agent, a radiotherapeutic agent, or an antibody drug.

Further, the form of the drug or composition may be suitable for: direct naked RNA injection method, direct liposome-encapsulated RNA injection method, direct protein or polypeptide-encapsulated RNA injection method, gold-coated RNA gene gun bombardment method, bacteria-carried plasmid-expressing RNA method, or virus-expressing RNA method.

Further, a form of the siRNA drug or composition is not particularly limited, and can be selected from the group consisting of a solid, a liquid, a gel, a semiliquid, and an aerosol.

Further, the MCM7 gene may be a human MCM7 gene.

The siRNA can be used to effectively inhibit the expression of the MCM7 gene and the synthesis of the MCM7 protein, thereby achieving the purpose of treating a disease including a tumor.

In a fourth aspect of the present disclosure, a method for inhibiting the expression of the MCM7 gene is provided, including: administering the siRNA of the present disclosure or the drug or composition of the present disclosure to an individual in need.

In a fifth aspect of the present disclosure, a method for preventing or treating a tumor/cancer is provided, including: administering the siRNA of the present disclosure or the drug or composition of the present disclosure to an individual in need. The tumor may show slowed or stopped growth, shrink, or die due to the inhibition of the expression of the MCM7 gene.

The present disclosure has the following beneficial effects:
In the present disclosure, the 9th base from the 5' terminus of the sense strand of the siRNA-1 can be changed, such that the sense strand and the antisense strand form an incomplete complementary pairing and the thermodynamic properties of the entire double-stranded RNA are changed, which improves the efficiency of the antisense strand to participate in the interference of RNA with a complex protein and thus improves the efficiency of the siRNA-1 to inhibit the target MCM7 gene.

The present disclosure provides a siRNA targeting the MCM7 gene, and compared with the conventional gene knockout technology, the present disclosure involves simple operation and a short test period. The siRNA shows an inhibitory effect as high as 90% or more at mRNA and protein levels, with extremely-high inhibition efficiency and prominent specificity. Moreover, the siRNA can effectively inhibit the DNA replication, proliferation, and colony formation ability of cancer cells, which is of great significance for the development of new anti-cancer gene drugs and the improvement of the cancer treatment effects, and has significant clinical application prospects and economic value.

### BRIEF DESCRIPTION OF THE DRAWINGS

FIG. 1 shows the silencing effects of the MCM7-specific siRNA-1 on the MCM7 mRNA levels in HepG2 and Hep3B liver cancer cells.
FIG. 2A and FIG. 2B show the silencing effects of the MCM7-specific siRNA-1 on the MCM7 protein levels in HepG2 and Hep3B liver cancer cells, respectively, where β-actin is an internal reference protein.

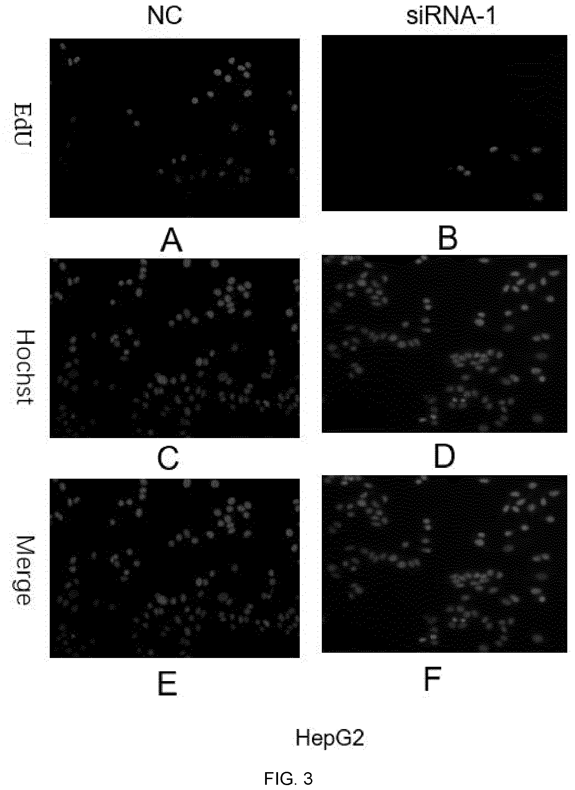

FIG. 3 shows fluorescence microscopy images of EdU-positive cells among HepG2 liver cancer cells whose DNA replication is inhibited by siRNA-1, where FIG. 3A, FIG. 3C, and FIG. 3E are a fluorescence microscopy image of EdU-positive cells, a Hochst-stained nuclear DNA image, and an overlay graph of the fluorescence microscopy image of EdU-positive cells and the Hochst-stained nuclear DNA image, respectively, after the negative control NC was used to transfect HepG2 liver cancer cells; and FIG. 3B, FIG. 3D, and FIG. 3F are a fluorescence microscopy image of EdU-positive cells, a Hochst-stained nuclear DNA image, and an overlay graph of the fluorescence microscopy image of EdU-positive cells and the Hochst-stained nuclear DNA image, respectively, after siRNA-1 was used to transfect HepG2 liver cancer cells.
FIG. 4 is a statistical chart of proportions of HepG2 cells positive for EdU incorporation.
FIG. 5A to FIG. 5E show growth curves of HepG2 liver cancer cells, Hep3B liver cancer cells, SGC-7907 gastric cancer cells, PC3 prostate cancer cells, and MCF7 breast cancer cells whose proliferation was inhibited by siRNA-1, respectively.
FIG. 6 shows the colony formation of various cancer cells inhibited by siRNA-1, where FIG. 6A to FIG. 6E show quantitative charts of the number of cancer cell colonies after the HepG2 liver cancer cells, Hep3B liver cancer cells, SGC-7907 gastric cancer cells, PC3 prostate cancer cells, and MCF7 breast cancer cells, respectively, were transfected with siRNA-1 or the negative control NC.
FIG. 7 shows the colony formation ability of various cancer cells inhibited by siRNA-1, where FIG. 7A to FIG. 7E show the proportion of the total cancer cell colony area in the total plate area after the HepG2 liver cancer cells, Hep3B liver cancer cells, SGC-7907 gastric cancer cells, PC3 prostate cancer cells, and MCF7 breast cancer cells, respectively, were transfected with siRNA-1 or the negative control NC.

### DETAILED DESCRIPTION OF THE EMBODIMENTS

The technical solutions in the present disclosure will be clearly and completely described below with reference to examples, but are not limited thereto.

Unless otherwise specified, the reagents and instruments used in the following examples are known in the art and are commercially available; and the experimental methods used are all conventional methods. Those skilled in the art can implement the solutions without any doubt and obtain corresponding experimental results according to the experimental contents described in the examples.

### Example 1 siRNA design

According to the basic principle of a siRNA target sequence, a siRNA sequence (with each strand of 21 nucleotides) expressed by a human MCM7 gene transcript (NM_001278595.1) was designed and synthesized, that is, the sense strand and antisense strand of the siRNA had the following base sequences:
sense strand of siRNA-1: 5'-GUGGAGAAUUGACCUUAGA dTdT-3' (SEQ ID NO: 1), and
antisense strand of siRNA-1: 5'-UCUAAGGUCAGUUCUCCAC dTdT-3' (SEQ ID NO: 2).

The negative control RNA (NC) had the following base sequences:
sense strand: 5'-CUCUUAGCCAAUAUUCGCU dTdT-3' (SEQ ID NO. 3); and
antisense strand: 5'-AGCGAAUAUUGGCUAAGAGdTdT-3' (SEQ ID NO. 4).

Two deoxyribonucleotides (dT or dN) in a single-strand overhang structure were added to the 3' termini of the sense and antisense strands of the siRNA sequence of the present disclosure and the control RNA to enhance the stability of the siRNA *in vivo* and *in vitro* and prevent the siRNA from being degraded by nucleases.

The siRNA sequence of the present disclosure can be modified or changed at some or all of its nucleotide sites, as long as the binding and inhibition of the target are not affected.

### Example 2 Transfection of cells with siRNA

The Lipofectamine RNAiMax was used as a transfection reagent, and the transfection was conducted in accordance with steps specified in the operating instruction of Thermo Fisher Scientific.

The cell lines used were HepG2 (purchased from American Type Culture Collection (ATCC), ATCC HB-8065) and Hep3B liver cancer cell lines (purchased from American Type Culture Collection (ATCC), ATCC HB-8064), the SGC-7907 gastric cancer cell line (purchased from Shanghai Institutes for Biological Sciences), the PC3 prostate cancer cell line (purchased from American Type Culture Collection (ATCC), ATCC CRL-1435), and the MCF7 breast cancer cell line (purchased from American Type Culture Collection (ATCC), ATCC HTB-22).

The experimental methods which are not specified with particular conditions in the present disclosure are generally conducted under conventional conditions, such as conditions disclosed in Molecular Cloning: Experiment Guide (Sambrook et al., New York: Cold Spring Harbor Laboratory Press, 1989) or conditions recommended by manufacturers.

Transfection steps were as follows: the above-mentioned different cancer cell lines were each inoculated into a 12-well plate, and cultivated overnight at 37°C and 5% CO₂ until the cell confluency reached 40% to 50%. With reference to the operating instruction of Lipofectamine RNAiMax (Thermo Fisher Scientific), the siRNA prepared in the present disclosure and the negative control RNA (NC) were used to transfect different cells. After the transfection, the cells were collected, and the interference effect of siRNA-1 was tested through quantitative reverse-transcription polymerase chain reaction (qRT-PCR) and western blotting.

### Example 3 Detection of the inhibition of the expression of MCM7 mRNA by siRNA

Method: After the transfection, the cells were collected, RNA was extracted and subjected to reverse transcription, and then real-time fluorescence quantification PCR was conducted to detect the expression of MCM7 mRNA in cancer cells treated with siRNA-1.

The siRNA-1 prepared in the present disclosure and the negative control RNA (NC) were used separately to transfect the HepG2 and Hep3B liver cancer cell lines. Twenty-four hours after the transfection, the cells were collected, an appropriate amount of the cells was re-inoculated into a 6-well plate, and the cells were collected 72 hours later to extract total RNA. Reverse transcription was conducted, and real-time fluorescence quantification PCR was conducted to detect MCM7 mRNA.

### 1. Total RNA extraction

(1) Tumor cells were collected into a centrifuge tube and centrifuged at 800 rpm for 3 min, the resulting supernatant was discarded, and the resulting precipitate was washed once with PBS and then transferred to an EP tube.
(2) The EP tube was centrifuged at 800 rpm for 3 min, the resulting supernatant was discarded, 0.5 ml of TRIzol was added, the contents were repeatedly pipetted up and down to dissolve the tumor cells, and the resulting suspension was set at room temperature for 5 min to 10 min.
(3) Chloroform was added at an amount of 0.2 ml chloroform/ml TRIzol, and the resulting mixture was shaken vigorously for 15 seconds and then stood for 10 min at room temperature.
(4) The mixture was centrifuged for 15 min at 12,000 rpm and 4°C.
(5) After the centrifugation, the mixture was seen separated into three layers, including a phenol/chloroform layer, an intermediate protein layer, and an upper colorless aqueous phase from bottom to top; and RNA was in the upper aqueous phase.
(6) The upper aqueous phase was pipetted into a new EP tube, where the intermediate protein layer should not be pipetted.
(7) Pre-cooled isopropyl alcohol (IPA) was added at 0.5 ml/ml TRIzol, the EP tube was inverted up and down for thorough mixing, and the resulting mixture was set for 10 min at room temperature.
(8) The mixture was centrifuged for 10 min at 12,000 rpm and 4°C.
(9) The resulting supernatant was discarded, the resulting RNA precipitate was washed with 75% ethanol (which was prepared with 750 µl of absolute ethanol and 250 µl of DEPC water just before use), and the resulting mixture was centrifuged at 12,000 rpm and 4°C for 5 min.
(10) The resulting supernatant was discarded, and the resulting precipitate was air-dried in a clean bench for about 3 min (the RNA pellet was translucent).
(11) Fifteen µl to 20 µl of 1‰ DEPC water were added to dissolve the RNA precipitate, and the resulting solution was subjected to concentration and absorbance (optical density; OD) determination with an ultraviolet (UV) spectrophotometer, and stored at -70°C or used directly for reverse transcription.

### 2. Reverse transcription into cDNA

The total RNA was subjected to high-temperature pre-denaturation for 5 min and then subjected to instant freezing on ice. A reverse transcription reaction system was as follows:

| | |
|---|---|
| 5 × reverse transcription buffer | 5 µl |
| dNTP (10 nM) | 0.5 µl |
| RNase inhibitor (20 U/µl) | 1 µl |
| RNase (20 U/µl) | 3 µl |
| RNA template | 1 µg |
| Reverse transcription primer (500 nM) | 2 µl |
| RNase-free deionized water | making up to 25 µl |

Reaction conditions: 37°C for 15 min, 50°C for 5 min, 98°C for 5 min, and holding at 4°C. The synthesized cDNA could be used immediately for downstream experiments or stored in a -20°C freezer.

### 3. Real-time fluorescence quantification PCR

A reversely-transcribed cDNA sample was diluted in an appropriate ratio, and then prepared with THUNDERBRID SYBR qPCR Mix into the following PCR reaction system:

| | |
|---|---|
| 2 ^{∗}Master Mix | 4.5 µl |
| upstream primer | 0.4 µl |
| downstream primer | 0.4 µl |
| template cDNA (five-fold diluted) | 4.7 µl |

The upstream primer sequence was 5'-GTGAAGGATCCTGCGACACA-3' (SEQ ID NO. 5);
the downstream primer sequence was 5'-ACACGCGTTCTTTTGTTCCG-3' (SEQ ID NO. 6);
the internal reference upstream primer sequence was 5'-AGAAGAGCTACGAGCTGCCTGACG-3' (SEQ ID NO. 7); and
the internal reference downstream primer sequence was 5'-GGACTCCATGCCCAGGAAGGAA-3' (SEQ ID NO. 8).

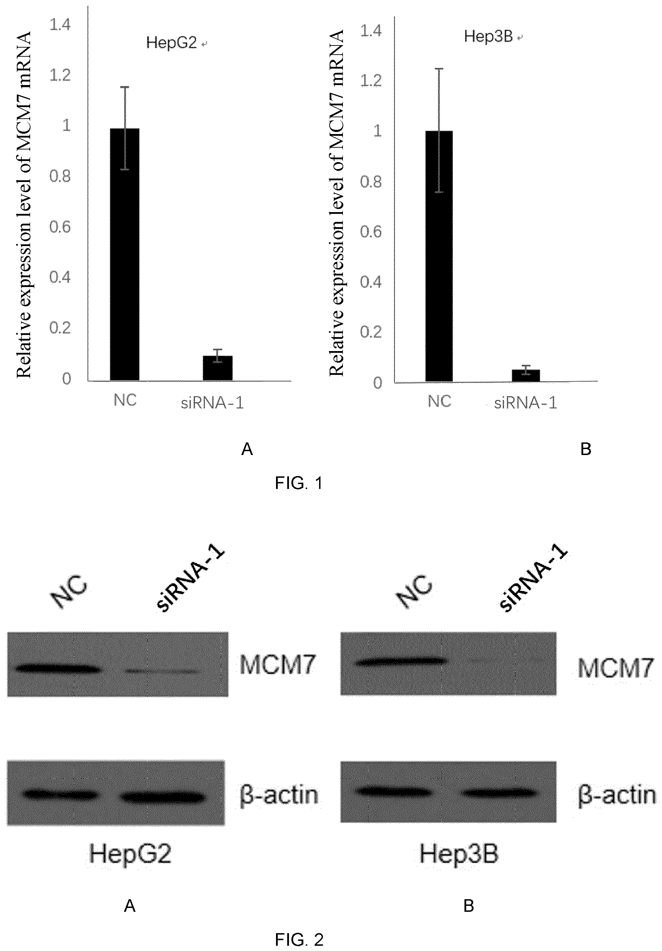

Results: FIG. 1A and FIG. 1B show that, compared with the control group NC, siRNA-1 can effectively inhibit the expression of MCM7 mRNA in HepG2 and Hep3B liver cancer cells after transfecting the cancer cells, with a silencing effect of 90% or more.

### Example 4 Detection of the inhibition of the expression of the MCM7 protein by siRNA

Method: The siRNA-1 prepared in the present disclosure and the negative control RNA (NC) were separately used to transfect the HepG2 and Hep3B liver cancer cell lines. Twenty-four hours after the transfection, the cells were collected, an appropriate amount of the cells was re-inoculated into a 12-well plate, and the cells were collected 72 hours later for western blotting.
1. The medium was removed, an appropriate amount of 2 × laemmli buffer was added, and the 12-well plate was gently shaken for cell lysis; the lysate was then collected into a PE tube, and the PE tube was rubbed against the holes of a PE tube rack to shatter DNA, and then boiled at 95°C for 2 min.
2. The boiled denatured sample was subjected to polyacrylamide gel electrophoresis (PAGE) and western blotting, and the polyvinylidene fluoride (PVDF) membrane with the proteins was blocked with 5% skimmed milk for 0.5 hours at room temperature.
3. An appropriate primary antibody (mouse anti-human MCM7 monoclonal antibody (mAb), Santa Cruz Biotechnology) was diluted at an appropriate ratio, and then incubated overnight with the PVDF membrane at 4°C.
4. The next day, the PVDF membrane was washed 3 times with TBST, 10 min each time.
5. An HRP-labeled anti-mouse IgG secondary antibody (Pierce) corresponding to the primary antibody host species was diluted at an appropriate ratio, and incubated for 1 hour with the PVDF membrane at room temperature.
6. The PVDF membrane was washed 3 times with TBST, 10 min each time.
7. An electrochemiluminescence (ECL) solution was used to develop the signals to detect the expression of the MCM7 protein in tumor cells.

Results: FIG. 2A and FIG. 2B show that, compared with the control group NC, siRNA-1 can effectively inhibit the expression of the MCM7 protein in HepG2 and Hep3B cells after transfecting the cancer cells, with an inhibition effect of 90% or more.

### Example 5 Inhibition of DNA replication of cancer cells by siRNA

Method: The siRNA-1 prepared in the present disclosure and the negative control RNA (NC) were separately used to transfect the HepG2 and Hep3B liver cancer cell lines. Twenty-four hours after the transfection, the cells were collected, and an appropriate amount of the cells was re-inoculated into a 96-well plate. Twelve hours later, the mimosine reagent was added to the cells, and the cells were incubated for 24 hours to synchronize the cells at the G₁/S phase junction.

Cells were released from the mimosine block by washing the cells three times with fresh medium at an interval of 3 min. The cells were cultured with fresh medium for 3.5 hours, 50 mmol/L 5-ethynyl-2'-deoxyuridine (EdU, a thymidine analog) was added, and the cells were further cultured for 0.5 hours. The cells were fixed, stained, and then observed under a fluorescence microscope, and the proportions of cells positive for EdU incorporation were determined.

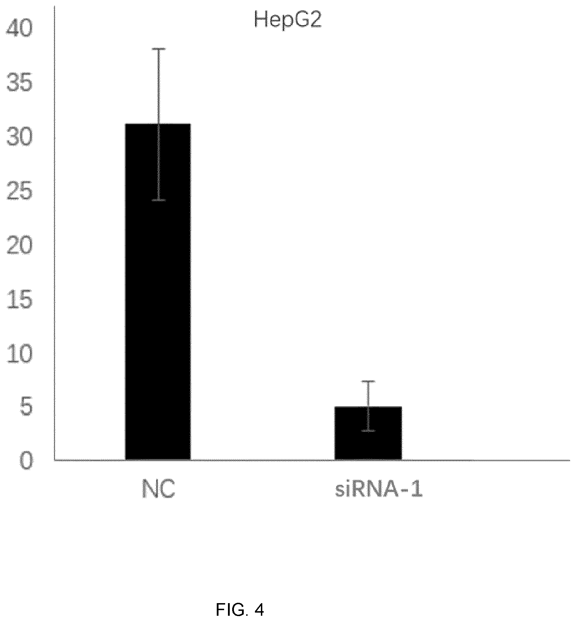

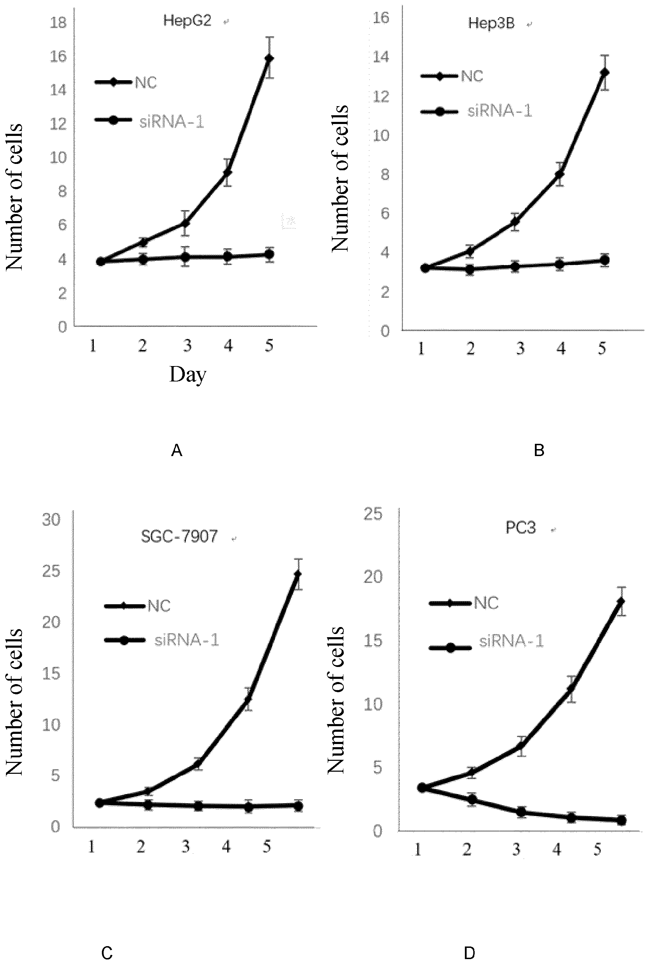

Results: FIG. 3 and FIG. 4 show that, compared with the negative control, after the HepG2 liver cancer cells were transfected with siRNA-1, the proportion of cells positive for EdU incorporation was significantly reduced, indicating that siRNA-1 can significantly inhibit DNA replication of cancer cells. Data of Hep3B cells were similar to that of HepG2 cells.

The fresh medium used was: Gibco RPMI 1640.

### Example 6 Inhibition of the proliferation of cancer cells by the MCM7 siRNA

Method: The siRNA-1 prepared in the present disclosure and the negative control RNA (NC) were separately used to transfect different cancer cell lines, and 24 hours after the transfection, the cells were collected. An appropriate amount of the cells were divided into five equal parts and re-inoculated into a 12-well plate, and counting was conducted for five days, where one well was selected for counting every day. Cell growth curves after the transfection were plotted.

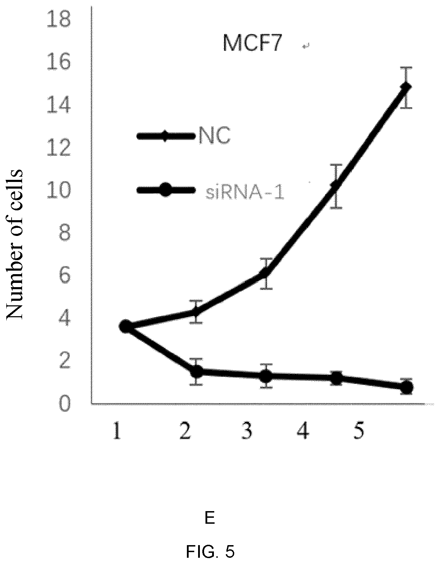

Results: FIG. 5 shows that the siRNA-1 can effectively inhibit the proliferation of HepG2 liver cancer cells, Hep3B liver cancer cells, SGC-7907 gastric cancer cells, PC3 prostate cancer cells, and MCF7 breast cancer cells.

### Example 7 Inhibition of the formation of cancer cell colonies by the MCM7 siRNA

Method: The siRNA-1 prepared in the present disclosure and the negative control RNA (NC) were separately used to transfect different cancer cell lines, and 24 hours after the transfection, the cells were collected. The cells were inoculated into a 6-well plate at a cell density of 0.4 × 10³ cells/well, cultured for 14 days, fixed with methanol, and then stained with crystal violet.

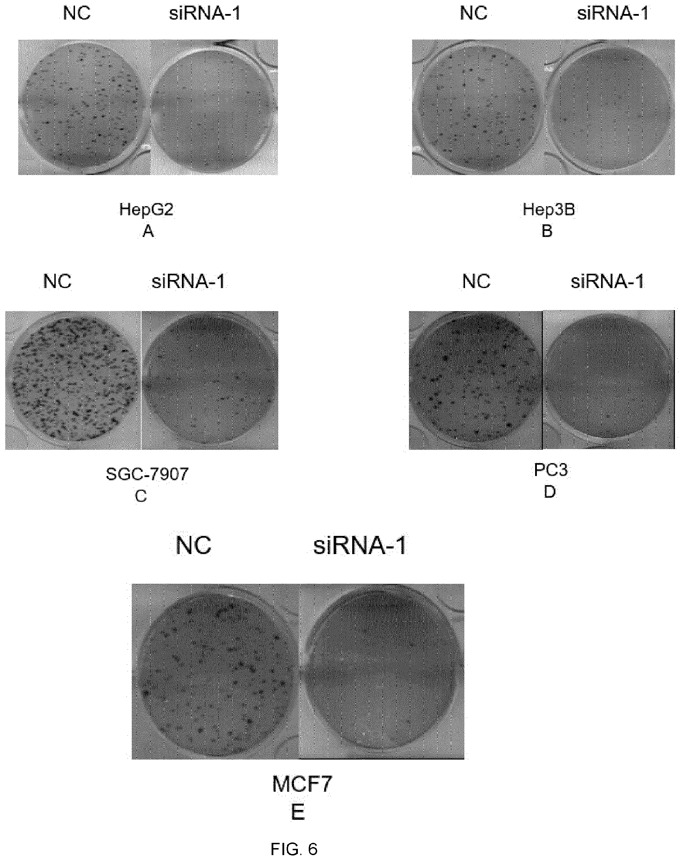

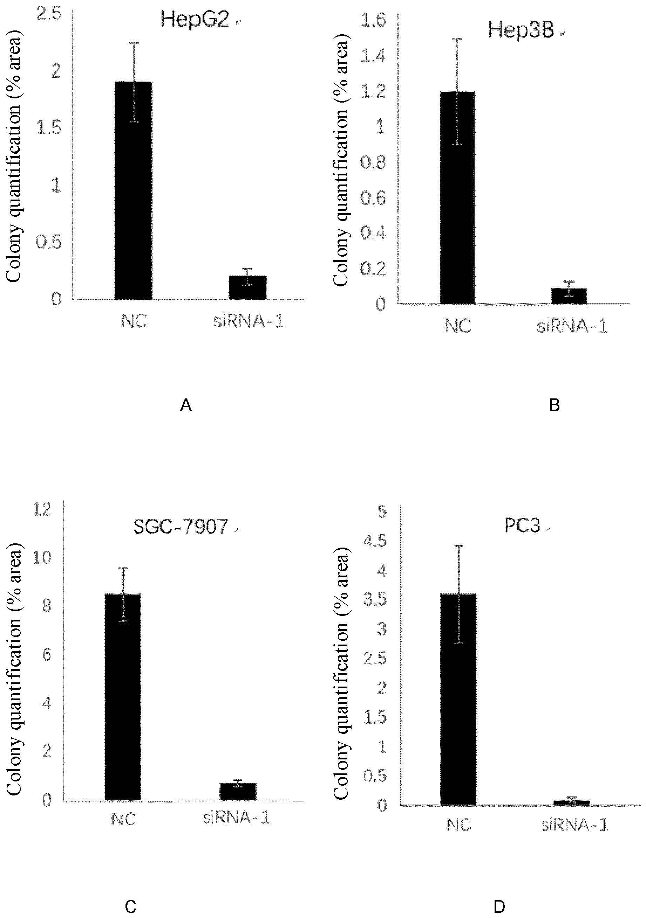

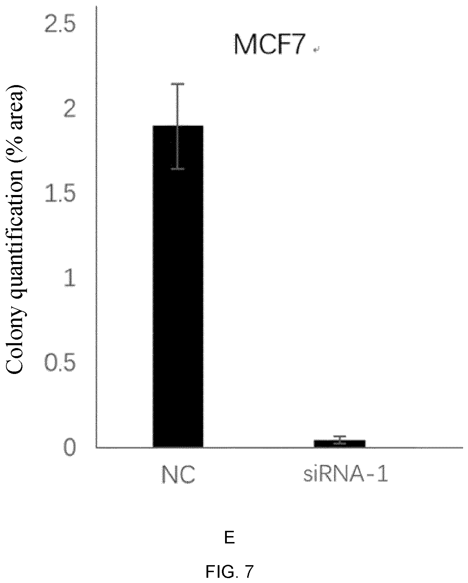

Results: As shown in FIG. 6 and FIG. 7, it can be seen from FIG. 6A to FIG. 6E that, compared with the negative control NC, after the cancer cells were transfected with siRNA-1, the number of cancer cell colonies was significantly reduced; and it can be seen from FIG. 7A to FIG. 7E that, compared with the negative control NC, after the cancer cells were transfected with siRNA-1, the proportion of the total colony area in the total plate area decreased. It can be known from the above that the siRNA can effectively inhibit the colony formation ability of HepG2 liver cancer cells, Hep3B liver cancer cells, SGC-7907 gastric cancer cells, PC3 prostate cancer cells, and MCF7 breast cancer cells.

### Example 8 Use of the MCM7 siRNA

The MCM7 siRNA of the present disclosure was used in the preparation of a compound for preventing or treating a tumor, and the tumor/cancer was selected from the group consisting of liver cancer, gastric cancer, prostate cancer, breast cancer, lung cancer, pancreatic cancer, cervical cancer, endometrial cancer, colorectal cancer, lung cancer, nasopharyngeal cancer, ovarian cancer, skin cancer, esophageal cancer, and brain tumor.

In summary, the siRNA of the present disclosure can effectively inhibit the expression of the MCM7 gene to reduce the synthesis of the MCM7 protein, and the siRNA shows an inhibitory effect of 90% or more, with extremely-high inhibition efficiency and prominent specificity. Moreover, the siRNA can effectively inhibit the DNA replication, proliferation, and colony formation ability of cancer cells, which is of great significance for the development of new anti-cancer gene compounds and the improvement of the cancer treatment effects, and has significant clinical application prospects and economic value.

The above examples are preferred implementations of the present disclosure. However, the implementations of the present disclosure are not limited by the above examples. Any change, modification, substitution, combination, and simplification made without departing from the spiritual essence and principle of the present disclosure should be an equivalent replacement manner, and all are included in the protection scope of the present disclosure.

## Claims

1. A small interfering RNA (siRNA), wherein the siRNA is capable of inhibiting the expression of the mini-chromosome maintenance 7 (MCM7) gene, and is composed of a sense strand and an antisense strand; and
the siRNA is selected from one of the groups consisting of:
siRNA-1: sense strand: 5'-GUGGAGAAUUGACCUUAGA-3' (SEQ ID NO: 9), and
antisense strand: 5'-UCUAAGGUCAGUUCUCCAC-3' (SEQ ID NO: 10);
a siRNA whose sense strand or antisense strand sequence has 80% or more homology and preferably 90% or more homology with the sense strand or antisense strand sequence of the siRNA-1; and
a siRNA whose one or more nucleotides are able to be modified or changed and which has the same or similar function and activity as or to the siRNA-1, wherein preferably, 2'-OH in one or more nucleotides is changed into 2'-methoxy and a central oxygen atom of phosphate is substituted by sulfur; or the sense strand or the antisense strand is added with cholesteryl.

2. The siRNA according to claim 1, wherein two deoxyribonucleotides dT or dN in a single-strand overhang structure need to be added to 3' termini of the sense strand and the antisense strand of the siRNA-1.

3. Use of the siRNA according to claim 1 or 2 in the preparation of a drug or a composition for preventing or treating a tumor/cancer.

4. The use according to claim 3, wherein the tumor/cancer is selected from the group consisting of liver cancer, gastric cancer, prostate cancer, breast cancer, lung cancer, pancreatic cancer, cervical cancer, endometrial cancer, colorectal cancer, lung cancer, nasopharyngeal cancer, ovarian cancer, skin cancer, esophageal cancer, and brain tumor.

5. A drug or composition for preventing or treating a tumor, comprising:
the siRNA according to claim 1 or 2; or
an expression system capable of expressing the siRNA according to claim 1 or 2, and preferably a short hairpin RNA (shRNA) expression system.

6. The drug or composition according to claim 5, wherein the tumor/cancer is selected from the group consisting of liver cancer, gastric cancer, prostate cancer, breast cancer, lung cancer, pancreatic cancer, cervical cancer, endometrial cancer, colorectal cancer, lung cancer, nasopharyngeal cancer, ovarian cancer, skin cancer, esophageal cancer, and brain tumor.

7. The drug or composition according to claim 5, further comprising:
a pharmaceutically acceptable carrier and/or adjuvant; and/or
other active ingredients for preventing or treating the tumor.

8. The drug or composition according to claim 7, wherein the pharmaceutically acceptable carrier and/or adjuvant comprise(s), but are/is not limited to a buffering agent, an emulsifying agent, a suspending agent, a stabilizer, a preservative, a physiological salt, an excipient, a filler, a coagulating agent, a conditioner, a surfactant, a diffusing agent, and a defoaming agent.

9. The drug or composition according to claim 7, wherein the other active ingredients for preventing or treating the tumor comprise a chemotherapeutic agent, a radiotherapeutic agent, or an antibody drug.

10. The drug or composition according to any one of claims 5 to 9, wherein the drug or composition is in any form selected from the group consisting of a solid, a liquid, a gel, a semiliquid, and an aerosol.
